# EUROPÄISCHE PATENTANMELDUNG

(11) **EP 1 872 767 A1**
(43) Veröffentlichungstag der Anmeldung: **02.01.2008**
(21) Anmeldenummer: 06013589.4
(22) Anmeldetag: 30.06.2006
(51) Int. Cl.: A61K 6/00, A61K 6/083

(54) **Polymerisierbares Dentalmaterial**

(71) Anmelder: Ernst Mühlbauer GmbH & Co.KG, 22547 Hamburg (DE)
(72) Erfinder: Lück, Rainer, Dr. sc., 25436 Tornesch (DE)
(74) Vertreter: Glawe, Delfs, Moll

(57) **Zusammenfassung**

Gegenstand der Erfindung ist ein wenigstens zwei Komponenten aufweisendes radikalisch polymerisierbares Dentalmaterial. Eine erste Komponente des Dentalmaterials enthält das Salz einer CH-aciden Verbindung und eine weitere Komponente einer Säure, deren Säurestärke größer ist als die der CH-aciden Verbindung in der ersten Komponente. Mindestens eine der Komponenten enthält Monomere. Bei der Mischung beider Komponenten wird das Salz der CH-aciden Verbindung der ersten Komponente durch die Säure der weiteren Komponente in ein CH-acides Molekül überführt, welches die radikalische Polymerisation der Monomere auslöst.

Die Erfindung erreicht neben den Vorteilen von CH-aciden Verbindungen in Dentalmaterialien auf der Basis einer reaktiven Harzmatrix eine lange Lagerstabilität und die Erhöhung des Anteils der reaktiven Harzmatrix im polymerisierbaren Dentalmaterialien.

## Beschreibung

Die Erfindung betrifft ein polymerisierbares Dentalmaterial und die Verwendung von Salzen einer CH-aciden Verbindung.

Chemisch härtende polymerisierbare Dentalmaterialien (in der Literatur häufig auch selbsthärtende oder autokatalysierte Dentalmaterialien genannt) enthalten polymerisierbare Monomere, deren Polymerisation durch primär gebildete Radikale ausgelöst wird. Die Bildung dieser Radikale erfolgt durch die Reaktion eines geeigneten Initiatormoleküls, das bei Raumtemperatur allein eine ausreichende Lagerstabilität besitzt, mit einem Coinitiator. Da diese Reaktion sofort nach Zusammentreffen von Initiator und Coinitiator beginnt, müssen zur Lagerung der Dentalmaterialien beide Komponenten des Initiatorsystems getrennt untergebracht werden. Es resultieren daher, im Unterschied zu lichthärtenden Materialien, bei denen die Radikale erst durch Bestrahlung mit der blauen Komponente des sichtbaren Lichtes gebildet werden, Mehr-Komponenten-Systeme. Die Komponenten werden erst unmittelbar vor der Verarbeitung des Materials miteinander in Kontakt gebracht und innig miteinander vermischt. Diese Vermischung kann dabei entweder von Hand mit einem Mischspatel oder durch selbstanmischenden Systeme (Doppelkartusche mit statischen oder dynamischen Mischkanülen) erfolgen.

Ein im Stand der Technik bei chemisch härtenden Dentalmaterialien am häufigsten verwendetes Initiatorsystem, besteht aus einem zumeist aromatischen Amin und einem organischen Peroxid, wie z.B. in DE-C-97 50 72 beschrieben. Die benötigten Radikale werden bei diesem System über eine Redoxreaktion zwischen Amin und Peroxid gebildet.

Ein wesentlicher Nachteil der Amin/Peroxidsysteme besteht in der allgemein schlechten Farbstabilität. Ursache dafür sind bei Parallel-, Neben- und Folgereaktionen gebildete Produkte der Initiatorkomponenten, die aufgrund ihrer Struktur häufig gefärbt sind. Solche gefärbten Verbindung können bei der Radikalbildung entstehen, die während der Lagerung der Pasten gebildet werden oder im ausgehärteten Material zum Beispiel durch Einwirkung von sichtbarem oder UV-Licht beobachtet werden (z.B. A. Schmidt: "Kaltpolymerisate: Ein Bericht über ihre Eigenschaften, Einsatzmöglichkeiten und Vorteile", Dentallabor 11 [1970] S. 17-22). Dieser Nachteil kann bei den Amin/Peroxid-Systemen auch durch den durchaus üblichen Zusatz spezieller Licht- und UV-Stabilisatoren nicht behoben werden. Für hochästhetische Versorgungen sind diese Verfärbungen für den Patienten störend bzw. nicht akzeptabel. Daher wird für Versorgungen im Frontzahnbereich häufig auf sehr viel aufwendigere und teurere Keramik (Veneers, Kronen, Brücken, u.ä.) zurückgegriffen.

Ein weiterer Nachteil der Amin/Peroxid-Systeme besteht in der toxischen und allergieauslösenden Wirkung der Komponenten des Startersystems und deren Reaktions- und Abbauprodukten. Während des Härtungsprozesses kann eine direkte toxische Wirkung von diesen Komponenten ausgehen. Auch nach der Härtung können entsprechende nicht einpolymerisierte Moleküle durch den sauren Speichel ausgewaschen werden. Bei einer Reihe von Patienten resultieren allergische Reaktionen, die die Anwendung von Kunststoffen einschränken oder ausschließen. In Einzelfällen kann die toxische Wirkung einen anaphylaktischen (allergischen) Schock auslösen, der durchaus lebensbedrohliche Formen annehmen kann.

Weiterhin problematisch ist der Temperaturanstieg bei der Polymerisation aufgrund der exothermen Reaktionsprozesse. Amin/Peroxid gestartete Systeme polymerisieren vergleichsweise schnell und besitzen so am Gelpunkt bereits einen sehr hohen Vernetzungsgrad (Umsatz an Doppelbindungen), was eine relativ hohe Wärmemenge aus der exothermen Reaktion freisetzt. Hohe Temperaturmaxima sind die Folge. Eine zu hohe Temperatur kann aber zu Pulpaschädigungen bis hin zum Absterben des Zahnes führen.

Ein alternatives Initiatorsystem, das eine günstigere Temperaturentwicklung und eine deutlich bessere Farbstabilität besitzt, benutzt CH-acide Verbindungen in Kombination mit zweiwertigen Übergangsmetallionen und Chloridionen. Entsprechende CH-acide Verbindungen wurden intensiv von H. Bredereck und seinen Mitarbeitern untersucht (H. Bredereck et all: "Über Ch-Aktive Polymerisationsinitiatoren - XIII. Mitt. Polymerisationen und Polymerisationsinitiatoren", die Makromolekulare Chemie 92 [1966] S. 70-90; H. Bredereck et all: "Polymerisationen und Polymerisationsinitiatoren - 16. Einfluß von Thio-Gruppen in Barbitursäurederivaten auf die Polymerisationsauslösung von Methacrylsäure-methylester", die Makromolekulare Chemie 176 [1975] S. 1713-1723). Von den CH-aciden Verbindungen haben sich im Dentalbereich die Barbitursäurederivate als günstig erwiesen. Sie sind in hohen Ausbeuten und Reinheiten darstellbar, industriell verfügbar (Chemische Fabrik Berg GmbH, Mainthalstr. 3, D-06749 Bitterfeld) und erlauben durch ihre Reaktionskinetik die Realisierung interessanter Eigenschaften.

Die Synthese der Barbitursäurederivate ist z.B. aus E. Fischer und A. Dilthey: "Über c-Dialkylbarbitursäuren und über die Ureide der Dialkylessigsäuren", Ann. 335 [1904] S. 335) bekannt und beschreibt die alkalische Kondensation von Derivaten des Malonsäurediethylesters mit N-substituiertem Harnstoff in Natriumalkoholat. Die dabei erhaltenen Natriumsalze der Barbitursäurederivate werden anschließend durch die Zugabe einer Säure, z.B. von Salzsäure, in die Barbitursäurederivate überführt.

Bei dem auf Barbitursäure bzw. deren Derivaten basierenden Initiatorsystem müssen die Barbitursäurederivate von den polymerisierbaren Monomeren getrennt aufbewahrt werden. Dies liegt darin begründet, dass CH-acide Verbindungen wie die Derivate der Barbitursäure bereits ohne die Mitwirkung von Cu(II)- und Clorid-Ionen durch Autoxidation durch Luftsauerstoff Hydroperoxide bilden. Diese Hydroperoxide zerfallen unter Bildung von Radikalen, welche die Polymerisation der reaktiven Monomere initiieren, so dass es binnen kurzer Zeit zur spontanen Polymerisation kommt. Dieser spontane Polymerisationsprozess kann durch Zugabe von Stabilisatoren für kurze Zeit (im Bereich von wenigen Stunden) verzögert oder unterdrückt werden, nicht hingegen über einen längeren Zeitraum, wie es bei lagerstäbilen Systemen erwünscht ist.

Stand der Technik ist hier der Ersatz reaktionsfähiger Harze in der Initiatorpaste durch solche, die unter dentalen Bedingungen durch CH-acide Verbindungen nicht zur Polymerisation gebracht werden können, oder durch Verbindungen, die keine Doppelbindungen enthalten (z.B. Polyethylenglycol).

Die erforderliche räumliche Trennung von polymerisierbaren Monomeren und CH-aciden Barbitursäurederivaten begrenzt den Anteil der polymerisierbaren Monomere im Dentalmaterial. Für fließfähige Materialien, die vorrangig aus herkömmlichen Doppelkartuschensystemen appliziert werden, kann die Zugabe nicht reaktiver Monomerer durch die Verringerung der Zugabe dieser Pastenkomponente (Mischungsverhältnisse von 2:1, 4:1 und 10:1) zumindest vermindert werden. Aus diesen Gründen werden Barbitursäurederivate in Verbindung mit Cu²⁺ und Cl⁻ derzeit ausschließlich bei den fließfähigen automatisch dosierenden und anmischenden provisorischen Kronen- und Brückenmaterialien eingesetzt. Allerdings wirkt auch die geringere Menge durch die Initiatorpaste zugesetzter nicht polymerisierender Monomere als Trennmittel, was zu einer Verschlechterung der mechanischen Eigenschaften (Druckfestigkeit, Biegefestigkeit, Härte usw.) und zu einer Zunahme der Schmierschicht führt.

Hochviskose Materialien, wie z.B. modellierbare chemisch härtender Füllungskomposite sind aus Kartuschensystemen nicht ausbringbar und nicht automatisch anmischbar. Sie werden in Drehspritzen oder Dosen untergebracht und von Hand angemischt. Die Dosierung erfolgt durch den Zahnarzt ausschließlich nach Augenmaß. Dosierhilfen haben sich bisher nicht durchgesetzt. Da gleiche Materialialmengen besser einschätzbar sind, als z.B. 10:1 werden diese Materialien ausschließlich im Mischungsverhältnis von 1:1 angewendet. Dieses Mischungsverhältnis ist vom Zahnarzt besser einschätztbar, als ungleiche Verhältnisse und führt so zu wesentlich kleineren Dosierfehlern und damit zu besseren Materialqualitäten. Obwohl die CH-aciden Barbitursäurederivate zu wesentlich besseren Farbstabilitäten führen, als Amin/Peroxid-Systeme, bleiben diese Systeme Füllungs- und Verblendmaterialien wegen der hohen Zugabe nicht polymerisierender Monomere und der damit einhergehenden Verschlechterung der mechanischen Eigenschaften bisher verschlossen.

Der vorliegenden Erfindung liegt die Aufgabe zu Grunde, ein Initiatorsystem für polymerisierbare Dentalmaterialien zu schaffen, dass die aus dem Stand der Technik bekannten Nachteile vermeidet, und darüber hinaus auch in 1:1 dosierenden Materialien wie Füllungs- und Verblendkunststoffen einsetzbar ist.

Die Erfindung löst diese Aufgabe durch ein polymerisierbares Dentalmaterial aus wenigstens zwei Komponenten, dass die folgenden Komponenten umfasst:
Komponente 1 enthaltend
   a) das Salz einer CH-aciden Verbindung, wobei die CH-acide Verbindung eine radikalische Polymerisation auslösen kann,
Komponente 2 enthaltend
   b) eine Säure, deren Säurestärke größer ist als die der in der in Komponente 1 als Salz vorliegenden CH-aciden Verbindung,
wobei mindestens eine der Komponenten des polymerisierbaren Dentalmaterials radikalisch polymerisierbare Monomere enthält.

Kern der Erfindung ist, das im Unterschied zu den Startersystemen basierend auf CH-aciden Verbindungen aus dem Stand der Technik eine Vorstufe des aktiven Startermoleküls, nämlich ein Salz der CH-aciden Verbindung, eingesetzt wird. Die CH-acide Verbindung wird erst nach der Zugabe einer Säure, deren Säurestärke größer ist als die der als Salz vorliegenden CH-aciden Verbindung gemäß der Regel "Salz einer schwachen Säure + starke Säure ergibt Salz einer starken Säure + schwache Säure", freigesetzt und kann anschließend als Startermolekül für den Polymerisationsprozess der Monomere fungieren.

Die Erfindung hat erkannt, dass im Unterschied zu den CH-aciden Verbindungen, wie sie in Startersystemen des Standes der Technik verwendet werden, das Salz der CH-aciden Verbindung auch über längere Zeiträume lagerstabil ist. Damit wird die Initiatoraktivität für die Polymerreaktion der polymerisierbaren Monomere auch bei längerer Lagerung der Komponenten des polymerisierbaren Dentalmaterials sichergestellt. Gemäß einer bevorzugten Ausführungsform der Erfindung sind die Pasten, aus denen das polymerisierbare Dentalmaterial angemischt wird, und insbesondere die das Salz der CH-aciden Verbindung enthaltende Paste, mehr als 3 Monate, vorzugsweise mehr als 6 Monate, besonders bevorzugt mehr als 24 Monate farb- und/oder lagerstabil ist.

Die Erfindung hat des Weiteren erkannt, dass man ausgehend von dem erfindungsgemäßen Startersystem, gemäß einer bevorzugten Ausführungsform der Erfindung in beide Komponenten des Dentalmaterials polymerisierbare Monomere einbringen kann. Damit kann die eingangs erläuterte unerwünschte Beschränkung der Menge an polymerisierbaren Monomeren im polymerisierbaren Dentalmaterial, d. h. der Anteil der Polymermatrix im Dentalmaterial, aufgehoben werden. Daraus ergeben sich auch vorteilhafte mechanische Eigenschaften des polymerisierten Dentalmaterials, da die Menge der nicht reaktiven Monomere (z.B. Monomere mit nicht reaktiven Doppelbindungen oder Verbindungen, die keine Doppelbindungen enthalten), die üblicherweise aus Gründen der Handhabbarkeit der Initiatorpaste zugefügt werden (z. B. zur Einstellung pastöser Eigenschaften um die Paste in Kartuschensystemen verwenden zu können), verringert bzw. ganz darauf verzichtet werden kann. Es ist bekannt, das nicht einpolymerisierende Harze oder Füllstoffe in den Basis- und Initiatorpasten wie Trennmittel wirken und je nach Gehalt die mechanischen Eigenschaften nachteilig beeinflussen. Dieser Effekt wird bei der Verwendung des erfindungsgemäßen Startersystems weitestgehend vermieden, bei dem gemäß einer bevorzugten Ausführungsform der Erfindung die Monomere beider Komponenten bei deren Mischung radikalisch polymerisieren.

Erfindungsgemäß bevorzugte radikalisch polymerisierbare Monomere sind aus der Gruppe der Acrylsäureester und Methacrylsäureester ausgewählt.

Das erfindungsgemäße polymerisierbare Dentalmaterial kann die Komponente 1 in einer ersten Paste und die Komponente 2 in einer zweiten Paste enthalten und in einem vorgesehenen Mischungsverhältnis beider Pasten von 1:10 oder größer, vorzugsweise 1:4 oder größer, weiter vorzugsweise 1:2 oder größer, besonders bevorzugt 1:1 angemischt werden.

Als Salz der CH-aciden Verbindung der Komponente 1 sind insbesondere Salze der α-Benzoyl-proprionitrile, α-Cyancarbonsäureester und -amide, cyclischer β-Oxonitrile, β-Diketone, cyclischer β-Diketone, cyclischer β-Oxocarbonsäureester, cyclischer β-Oxo-lactone, Malonsäure, Malonsäurederivate, Pyrazolderivate, Barbitursäure oder Barbitursäurederivate geeignet.

Das Salz der CH-aciden Verbindung ist vorzugsweise ein Salz ausgewählt aus der Gruppe bestehend aus einwertigen und zweiwertigen Salzen der Alkali- und Erdalkaliionen. Das Salz der CH-aciden Verbindung kann beispielsweise ein Natriumsalz sein.

Als Säure der Komponente 2 im Sinne vorliegender Erfindung kann eine organische oder anorganische Säure verwendet werden, sofern deren Säurestärke größer ist als die der in der Komponente 1 als Salz vorliegenden CH-aciden Verbindung.

Als anorganische Säuren kommen nicht oxydierende Säuren wie z.B. Salzsäure oder Phosphorsäure in Betracht.

Besonders geeignete organische Säuren sind Monocarbonsäuren, ausgewählt aus der Gruppe bestehend aus Ameisensäure, Essigsäure und Benzoesäure bzw. Derivate dieser Säuren oder Dicarbonsäuren, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure bzw. Derivate dieser Säuren oder Tricarbonsäuren, ausgewählt aus der Gruppe bestehend aus Hemimellithsäure, Trimellithsäure, Trimesinsäure, Agaricinsäure, Citronensäure, 1,2,3-Propantricarbonsäure bzw. Derivate dieser Säuren oder Multicarbonsäuren, ausgewählt aus der Gruppe bestehend aus Pyromellithsäure und Mellithsäure bzw. Derivate dieser Säuren oder Polycarbonsäuren, ausgewählt aus der Gruppe bestehend aus Polyacrylsäure und Polymethacrylsäure bzw. Derivate dieser Säuren.

Der sich auf das Dissoziationsgleichgewicht in einem wässrigen Medium beziehende pKₐ-Wert kann dabei als ein Maß für die CH-Acidität zumindest mit herangezogen werden. Eine Auswahl an organischen Säuren im Sinne vorliegender Erfindung, deren pKₐ-Wert kleiner als der pKₐ-Wert der Barbitursäure (4,01) ist, sind beispielsweise 2,5-Dihydroxybenzoesäure (2,97), Fumarsäure (3,03), Maleinsäure (1,83), Phtalsäure (2,89), Salicylsäure (2,97), 2,4,6-Trihydroxybenzoesäure (1,68) und Zimtsäure (3,89).

Das erfindungsgemäße polymerisierbare Dentalmaterial kann in mindestens einer der Komponenten des polymerisierbaren Dentalmaterials 2-wertige Ionen, vorzugsweise Cu²⁺-Ionen und/oder Chlorid-Ionen enthalten, die den Polymerisationsprozess steuern und beschleunigen. Die fakultativ zum Startersystem gehörenden Cu²⁺- und Cl⁻ -Salze liegen vorzugsweise in der Basispaste (Komponente 1) des anspruchsgemäßen polymerisierbaren Dentalmaterials vor, können aber je nach Erfordernis auch der Inititiatorpaste (Komponente 2) zugesetzt werden.

Das erfindungsgemäße Dentalmaterial kann in mindestens einer der Komponenten Füllstoffe enthalten. Bei den erfindungsgemäß eingesetzten Füllstoffen handelt es sich bevorzugt um nano- und/oder mikroskalige (teilweise röntgenopake) Füllstoffe, vorzugsweise um Glaspulver, Glaskeramikpulver, Metall-, Halbmetall- oder Mischmetalloxide, Silikat-, Nitrid-, Sulfat-, Titanat-, Zirkonat-, Stannat-, Woframat-, Siliciumdioxid- Verbindungen oder eine Mischung aus diesen Verbindungen oder sphärische Füllstoffe, Quarzpulver oder eine Mischung aus diesen Pulvern oder gefüllte oder ungefüllten Splitterpolymerisate und/oder Perlpolymerisate. Bei den erfindungsgemäß eingesetzten nanoskaligen Füllstoffen handelt es sich besonders bevorzugt um Siliziumdioxid, Aluminiumoxid, Zirkondioxid, Titandioxid, Zinkoxid, Zinndioxid, Ceroxid, Aluminium-Silizium-Oxide, Silizium-Zink-Oxide, Silizium-Zirkon-Oxide, Eisenoxide und deren Mischungen mit Siliziumdioxid, Indiumoxide und deren Mischungen mit Siliziumdioxid und/oder Zinndioxid, Bornitrid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Der Füllstoff kann gemäß einer bevorzugten Ausführungsform der Erfindung ein oberflächenmodifizierter Füllstoff, vorzugsweise eine organisch oberflächenmodifizierter Füllstoff sein. Der Füllstoff kann nach seiner Oberflächenmodifikation, beispielsweise einer Silanisierung, auf seiner Oberfläche funktionelle Gruppen, beispielsweise reaktive Methacrylatgruppen besitzen, die mit den Monomeren chemisch, vorzugsweise radikalisch, reagieren können oder eine hohe Affinität zu der aus den Monomeren gebildeten Polymermatrix aufweisen.

Das erfindungsgemäße Dentalmaterial kann zur Einstellung bestimmter Eigenschaften zusätzlich so genannte Additive bzw. Modifikatoren enthalten. Die Allgemeinheit nicht einschränkend sind nachfolgend einige Beispiele genannt: anorganische und/oder organische Farbpigmente bzw. Farbstoffe, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).

Das erfindungsgemäße Dentalmaterial kann für die prothetische, konservierende und präventive Zahnheilkunde verwendet wenden. Ohne Anspruch auf Vollständigkeit seien stellvertretend einige Anwendungsbeispiele genannt:
Füllungsmaterial, Stumpfaufbaumaterial, Befestigungsmaterial, provisorisches und permanentes Kronen- und Brückenmaterial, Bonding-Materialien, zahntechnischer Werkstoff zur Herstellung von Inlays, Onlays, Verblendschalen, künstlichen Zähnen, Modellmaterialien und Fissuren- und Wurzelkanal-Versiegelungsmaterial.

Die Erfindung wird nachfolgend ohne Einschränkung der Allgemeinheit anhand von Ausführungsbeispielen erläutert.

### Beispiel 1: Synthese des Natriumsalzes der 1,3,5-Trimethylbarbitursäure

Die Synthese wurde von A.C.Cope et all.: "1,3-Dimethyl-5-alkyl Barbituric Acids": J.Amer.Chen.Soc. 63 365 (1941)beschrieben. 0,1 mol (=17,420g) Methylmalonsäurediethyl-ester wurden zu 97,214 g 21%-ige Natriumalkoholatlösung in Ethanol (= 0,3 mol Natriumalkoholat) gegeben und beide Komponenten innig miteinander vermischt. Dabei entstehen Natrium-methylmalonsäure-diethylester und Ethanol. Anschließend wurde 0,1 mol (= 8,811 g) N, N-Dimethylharnstoff in 15 ml Ethanol (p.a.) gelöst und zur Lösung langsam zugetropft, wobei das Natriumsalz der 1,3,5-Trimethylbarbitursäure entsteht. Anschließend wurde der Ansatz 11,5 Stunden unter Rückfluß gekocht. 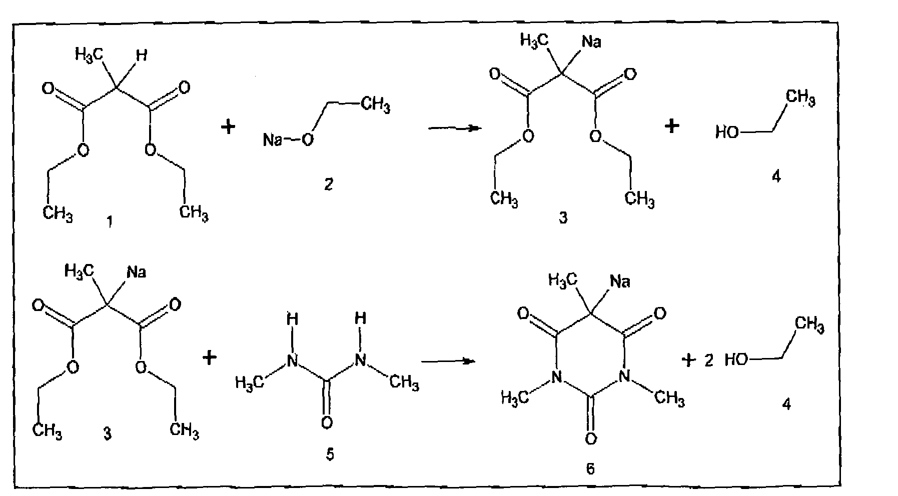
1 = Methylmalonsäure-diethylester
2 = Natriumalkoholat
3 = Natrium-methylmalonsäure-diethylester
4 = Ethanol
5 = N,N-Dimethylharnstoff
6 = 1,3,5-Trimethylbarbitursäure-Natriumsalz

Die Lösung wurde am Rotationsverdampfer bis zur Trockne eingeengt und der Rückstand mit 100 ml deionisiertem Wasser aufgenommen. Danach wurde 5 Mal mit je 20 ml Ether ausgeschüttelt. Die wässrige Phase wurde am Rotationsverdampfer erneut bis zur Trockne eingeengt und anschließend in eine Filter-nutsche überführt. Hier wurde der Rückstand mit Isopropanol gewaschen, bis das abgesaugte Isopropanol keine Färbung mehr aufwies. Da der Feststoff immer noch leicht bräunlich gefärbt war, wurde mit wenig Ethanol (p.a.) gewaschen, bis auch das Ethanol keine Färbung mehr aufwies. Die Reinheit wurde mittels HPLC mit 99,59% bestimmt. Die Ausbeute betrug 65,30%.

### Beispiel 2: Herstellung und Lagerstabilität der Initiatorpaste

Es wurden drei unterschiedliche Initiatorpasten hergestellt und auf ihre Lagerstabilität bei Raumtemperatur und bei 40°C untersucht:
- Paste 1:: Paste mit Polyethylenglycol mit mittlerem Molekulargewicht von 400 g/mol (PEG 400) als unreaktive Harzkomponente und mit Dentalglas gefüllt, das mit methacrylatgruppenfreiem Silan oberflächenbehandelt war und 1,3,5-Trimethylbarbitursäure als Startermolekül enthielt.
- Paste 2:: Paste mit Methacrylaten und mit Dentalglas gefüllt, das mit Methacrylatgruppen tragendem Silan oberflächenbehandelt war und 1,3,5-Trimethylbarbitursäure als Startermolekül enthielt.
- Paste 3:: Paste mit Methacrylaten und mit Dentalglas gefüllt, das mit Methacrylatgruppen tragendem Silan oberflächenbehandelt war und das Natriumsalz der 1,3,5-Trimethylbarbitursäure als Startermolekül enthielt.

Die folgende Tabelle zeigt die Rezepturen der Pasten. Dabei war die zugegebene Startermolekülmenge so berechnet, dass alle drei Pasten gleiche Molzahlen enthielten.

| Bestandteil | Paste 1 | Paste 2 | Paste 3 |
|---|---|---|---|
| | Gew.% | Gew.% | Gew.% |
| | | | |
| Bis-GMA | - | 37,2179 | 36,9790 |
| TEDMA | - | 16,7211 | 16,6138 |
| PEG 400 | 53,9390 | - | - |
| Aerosil R812 | 1,5000 | 1,5000 | 1,4904 |
| Dentalglas sil. ohne Methacrylatgruppen | 39,9350 | - | - |
| Dentalglas sil. mit Methacrylatgruppen | - | 39,9350 | 39,6785 |
| 1,3,5-Trimethylbarbitursäure | 4,6260 | 4,6260 | - |
| 1,3,5-Trimethylbarbitursäure Natriumsalz | - | - | 5,2383 |

Um eine Aussage über die Lagerstabilität der Pasten zu erhalten, wurden die Pasten bei Raumtemperatur und bei 40°C eingelagert und in regelmäßigen Abständen auf polymerisierte Anteile untersucht. Während die Paste 2 bereits nach 90 min vollständig auspolymerisiert war, zeigen die Pasten 1 und 3 auch nach mehr als 6 Monaten noch keinerlei Anzeichen von Polymerisation. Das zeigt, dass auch Pasten mit reaktiven Monomeren und 1,3,5-Trimethylbarbitursäure als aktives Startermolekül nicht lagerstabil sind, während bei Einsatz des Natriumsalzes der 1,3,5-Trimethylbarbitursäure als Vorstufe des aktiven Startermoleküls 1,3,5-Trimethylbarbitursäure lagerstabile Pasten resultieren.

### Beispiel 3: Untersuchung der Reaktionsfähigkeit der Initiatorpasten aus Beispiel 2

Um die Reaktionsfähigkeit der im Beispiel 2 hergestellten Initiatorpasten zu untersuchen, wurden diese von Hand mit der Basispaste des Produktes Luxatemp Automix A2 der Fa. DMG Hamburg im Verhältnis 10 ; 1 Charge (10 Teile Basispaste, 1 Teil Initiatorpaste) angemischt und die Erhärtungszeit bestimmt.

Mit der Polyethylenglykolhaltigen Paste 1 begann die Erhärtung nach 1:40 min. und war nach 3:20 min. abgeschlossen. Das bedeutet, dass diese Paste lagerstabil und reaktionsfähig ist.

Bei Verwendung der Initiatorpaste 2 ergibt sich ein Erhärtungsbeginn von 1:50 min. die Erhärtung ist hier ebenfalls nach ca. 3:20 min. abgeschlossen. D.h. die Paste besitzt eine ausreichende Reaktivität, ist aber, wie Beispiel 2 zeigte, nicht lagerstabil. Sie ist daher als Initiatorpaste nicht einsetzbar.

Die Initiatorpaste 3, die reaktive Methacrylgruppen und das Natriumsalz der 1,3,5-Trimethylbarbitursäure enthält, erhärtete nach dem Anmischen mit der oben genannten Basis erwartungsgemäß nicht. Deshalb wurde dem angemischten System (0,6g Basispaste + 0,06g Initiatorpaste) 1 Tropfen 32%-ige Salzsäure zugesetzt. Nach 55 min. polymerisierte das Material.

### Beispiel 4: Reaktion der Initiatorpaste 3 aus Beispiel 2 mit Salzsäure bzw. einer Salzsäure enthaltenden Basispaste

Die Beispiel 2 das Natriumsalz der 1,3,5-Trimethylbarbitursäure und reaktive Methacrylatmonomere enthaltende Initiatorpaste 3 wurde mit einem Tropfen Salzsäure gemischt. Nach ca. 90 Minuten war die Paste ausgehärtet.

In einem zweiten Versuch wurde der Initiatorpaste 3 aus Beispiel 2 neben dem Tropfen Salzsäure eine reaktive Methacrylatmonomere enthaltende Basispaste im Verhältnis 1:1 zur Initiatorpaste zugemischt. Die erhaltene Paste polymerisierte nach 20 Minuten.

Die Versuche belegen, dass das Natriumsalz des Barbitursäurederivats *in situ* in die freie Säure nach Zugabe der Salzsäure überführt wird und nach Aufbau der CH-Acidität die Pasten infolge der sich anschließenden Autoxydation nach kurzer Zeit polymerisieren.

### Beispiel 5: Reaktion der Initiatorpaste 3 aus Beispiel 2 mit organischen Säuren

Es wurden eine Auswahl an organischen Säuren, deren pKₐ-Wert unter dem der Barbitursäure liegt, auf Ihre Fähigkeit geprüft, das Natriumsalz der Paste 3 gemäß Beispiel 2 in die freie 1,3,5-Trimethylbarbitursäure zu überführen. Als Maß für die Überführbarkeit diente die Erhärtungszeit des Pastengemisches. Die Konzentration der verwendeten organischen Säuren wurde so berechnet, dass sie äquimolar zu der in der Initiatorpaste eingesetzten Molzahl des Startermoleküls bei einer Anmischung von 10:1 war. Bei der Anmischung der Initiatorpaste mit den nachfolgend genannten organischen Säuren
2,5-Dihydroxybenzoesäure,
Fumarsäure,
Maleinsäure,
Phtalsäure,
Salicylsäure,
2,4,6-Trihydroxybenzoesäure und
Zimtsäure
war innerhalb von 30 Minuten eine Vergelung, d.h. Polymerisation, zu beobachten.

Beispielhaft sei dies nachfolgend für die Reaktivität der Initiatorpaste 3 aus Beispiel 2 nach Zugabe von Fumarsäure bzw. 2,5-Dihydroxybenzoesäure beschrieben.

0,5g der oben genannten Basispaste wurden mit 0,05g der Initiatorpaste 3 aus Beispiel 2 vermischt und 1 Tropfen in Hydroxyethylmethacrylat gelöste Fumarsäure (0,1g in 10 ml) zugegeben. Das Material polymerisierte nach 6 Minuten.

In einem weiteren Versuch wurde nun die Fumarsäurelösung in die Basispaste eingearbeitet. Dabei wurde ein Molverhältnis von Fumarsäure: 1,3,5-Trihydroxybarbitursäurenatriumsalz von 1:1 (nach Anmischung der Pasten im Verhältnis von 10:1) eingestellt. Das Material polymerisierte nach 19 Minuten.

In einem weiteren Versuch wurde die in Hydroxyethylmethacrylat besser lösliche 2,5-Dihydroxybenzoesäure eingesetzt. Dazu wurde 0,1g der 2,5-Dihydroxybenzoesäure in 1 ml Hydroxyethylmethacrylat gelöst. 1 Tropfen der Lösung (0,0248g) wurden in 0,5g der oben genannten Basispaste eingearbeitet. Anschließend wurden 0,05g der Initiatorpaste 3 aus Beispiel 2 zugegeben und gründlich durchmischt. Nach 9 min. war das Pastengemisch erhärtet.

## Patentansprüche

1. Polymerisierbares Dentalmaterial aus wenigstens zwei Komponenten, **dadurch gekennzeichnet, dass** es die folgenden Komponenten umfasst:
Komponente 1 enthaltend
c) das Salz einer CH-aciden Verbindung, wobei die CH-acide Verbindung eine radikalische Polymerisation auslösen kann,
Komponente 2 enthaltend
d) eine Säure, deren Säurestärke größer ist als die der in der in Komponente 1 als Salz vorliegenden CH-aciden Verbindung,
wobei mindestens eine der Komponenten des polymerisierbaren Dentalmaterials radikalisch polymerisierbare Monomere enthält.

2. Polymerisierbares Dentalmaterial nach Anspruch 1, **dadurch gekennzeichnet, dass** die Komponente 1 und die Komponente 2 radikalisch polymerisierbare Monomere enthalten.

3. Polymerisierbares Dentalmaterial nach Anspruch 2, **dadurch gekennzeichnet, dass** die Monomere in Komponente 1 mit den Monomeren in Komponente 2 bei der Mischung beider Komponenten radikalisch polymerisieren können.

4. Polymerisierbares Dentalmaterial nach einem der vorangehenden Ansprüche, **dadurch gekennzeichnet, dass** die Komponente 1 in einer ersten Paste und die Komponente 2 in einer zweiten Paste enthalten ist und dass das vorgesehene Mischungsverhältnis 1:10 oder größer, vorzugsweise 1:4 oder größer, weiter vorzugsweise 1:2 oder größer, besonders bevorzugt 1:1 beträgt.

5. Polymerisierbares Dentalmaterial nach Anspruch 4, **dadurch gekennzeichnet, dass** die Pasten mehr als 3 Monate, vorzugsweise mehr als 6 Monate, besonders bevorzugt mehr als 24 Monate farb- und/oder lagerstabil sind.

6. Polymerisierbares Dentalmaterial nach einem der vorangehenden Ansprüche, **dadurch gekennzeichnet, dass** das Salz der CH-aciden Verbindung der Komponente 1 ein Salz der α-Benzoyl-proprionitrile, α-Cyan-carbonsäureester und -amide, cyclischer β-Oxonitrile, β-Diketone, cyclischer β-Diketone, cyclischer β-Oxocarbonsäureester, cyclischer β-Oxo-lactone, Malonsäure, Malonsäurederivate, Pyrazolderivate, Barbitursäure oder Barbitursäurederivate ist.

7. Polymerisierbares Dentalmaterial nach einem der vorangehenden Ansprüche, **dadurch gekennzeichnet, dass** das Salz der CH-aciden Verbindung ein Salz ausgewählt aus der Gruppe bestehend aus einwertigen und zweiwertigen Salzen der Alkali- und Erdalkaliionen, vorzugsweise ein Natriumsalz ist.

8. Polymerisierbares Dentalmaterial nach einem der vorangehenden Ansprüche, **dadurch gekennzeichnet, dass** die Säure der Komponente 2 eine anorganische Säure, vorzugsweise Phosphorsäure und/oder Salzsäure ist.

9. Polymerisierbares Dentalmaterial nach einem der vorangehenden Ansprüche, **dadurch gekennzeichnet, dass** die Säure eine organische Säure ist.

10. Polymerisierbares Dentalmaterial nach Anspruch 9, **dadurch gekennzeichnet, dass** die organische Säure eine Monocarbonsäure, ausgewählt aus der Gruppe bestehend aus Ameisensäure, Essigsäure und Benzoesäure bzw. Derivate dieser Säuren oder eine Dicarbonsäure, ausgewählt aus der Gruppe bestehend aus Oxalsäure, Malonsäure, Succinsäure, Adipinsäure, Pimelinsäure, Azelainsäure, Sebacinsäure, Maleinsäure, Fumarsäure, Sorbinsäure, Phthalsäure und Terephthalsäure bzw. Derivate dieser Säuren oder eine Tricarbonsäure, ausgewählt aus der Gruppe bestehend aus Hemimellithsäure, Trimellithsäure, Trimesinsäure, Agaricinsäure, Citronensäure, 1,2,3-Propantricarbonsäure bzw. Derivate dieser Säuren oder eine Multicarbonsäure, ausgewählt aus der Gruppe bestehend aus Pyromellithsäure und Mellithsäure bzw. Derivate dieser Säuren oder eine Polycarbonsäure, ausgewählt aus der Gruppe bestehend aus Polyacrylsäure und Polymethacrylsäure bzw. Derivate dieser Säuren ist.

11. Polymerisierbares Dentalmaterial nach einem der vorangehenden Ansprüche, **dadurch gekennzeichnet, dass** die radikalisch polymerisierbaren Monomere ausgewählt sind aus der Gruppe bestehend aus Acrylsäureester und Methacrylsäureester.

12. Polymerisierbares Dentalmaterial nach einem der vorangehenden Ansprüche, **dadurch gekennzeichnet, dass** mindestens einer der Komponenten des polymerisierbaren Dentalmaterials 2-wertige Ionen, vorzugsweise Cu²⁺-Ionen, und/oder Ammonium-Ionen und/oder Chlorid-Ionen enthält.

13. Polymerisierbares Dentalmaterial nach einem der vorangehenden Ansprüche, **dadurch gekennzeichnet, dass** die Komponente 1 2-wertige Ionen, vorzugsweise Cu²⁺-Ionen, und/oder Ammonium-Ionen und/oder Chlorid-Ionen enthält.

14. Polymerisierbares Dentalmaterial nach einem der vorangehenden Ansprüche, **dadurch gekennzeichnet, dass** mindestens eine der Komponenten des polymerisierbaren Dentalmaterials ein Füllstoff ist, ausgewählt aus der Gruppe bestehend aus micro- und/oder nanoskaligen Füllstoffen, vorzugsweise Metall-, Halbmetall- oder Mischmetalloxide, Silikaten, Nitriden, Sulfaten, Titanaten, Zirkonaten, Stannaten, Wolframaten, Siliciumdioxiden oder einer Mischung aus diesen Verbindungen oder sphärischen Füllstoffen, Quarzpulvern, Glaspulvern, Glaskeramikpulvern oder eine Mischung aus diesen Pulvern oder gefüllten oder ungefüllten Splitterpolymerisaten und/oder Perlpolymerisaten.

15. Polymerisierbares Dentalmaterial nach Anspruch 14, **dadurch gekennzeichnet, dass** der Füllstoff ein oberflächenmodifizierter Füllstoff, vorzugsweise eine organisch oberflächenmodifizierter Füllstoff ist.

16. Polymerisierbares Dentalmaterial nach Anspruch 15, **dadurch gekennzeichnet, dass** der oberflächenmodifizierte Füllstoff auf seiner Oberfläche funktionelle Gruppen besitzt, die mit den Monomeren chemisch, vorzugsweise radikalisch, reagieren können oder eine hohe Affinität zu der aus den Monomeren gebildeten Polymermatrix haben.

17. Polymerisierbares Dentalmaterial nach Anspruch 16, **dadurch gekennzeichnet, dass** der Füllstoff mit reaktiven Acrylat- oder Methacrylatgruppen tragendem Silan oberflächenmodifiziert ist.

18. Verwendung des polymerisierbaren Dentalmaterials nach einem der vorangehenden Ansprüche als Füllungsmaterial, Stumpfaufbaumaterial, Befestigungsmaterial, Bondingmaterial, provisorisches und permanentes Kronen- und Brückenmaterial, zahntechnischen Werkstoff zur Herstellung von Inlays, Onlays, Verblendschalen, künstlichen Zähnen, Modellmaterialien und Fissuren- bzw. Wurzelkanal-Versiegelungsmaterial.

19. Gehärtetes Dentalmaterial, erhältlich aus einem polymerisierbaren Dentalmaterial nach einem der Ansprüche 1 bis 17.

20. Verwendung von Salzen einer CH-aciden Verbindung als Komponente in radikalisch polymerisierbaren Dentalmaterialien.
